# EUROPEAN PATENT SPECIFICATION

(11) **EP 1 934 195 B1**
(45) Date of publication and mention of the grant of the patent: **25.02.2009**
(21) Application number: 05787303.6
(22) Date of filing: 10.09.2005
(51) Int. Cl.: C07D 309/30

(54) **METHOD FOR PRODUCING LACTONES**
VERFAHREN ZUR HERSTELLUNG VON LACTONEN
MÉTHODE DE SYNTHÈSE DE LACTONES

(43) Date of publication of application: 25.06.2008
(73) Proprietor: Ulkar Kimya Sanayii Ve Ticaret A.S., Besiktas 34353 Istanbul (TR)
(72) Inventor: ASLAN, Tuncer, 34353 Besiktas (TR); ÜNSAL, Serafettin, 34353 Besiktas - Istanbul (TR)
(74) Representative: Weller, Wolfgang
(86) International application number: PCT/EP2005/009740
(87) International publication number: WO 2007/028412

(56) References cited:
- EP-A- 0 247 633
- US-A- 5 280 126
- US-A1- 2002 161 239

## Description

This invention relates to a method for producing lactones of the following formula
wherein

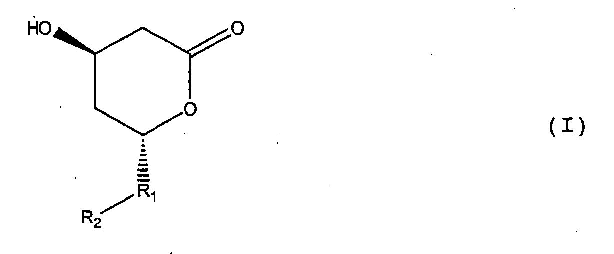

### R₁ is -CH₂-CH₂- or -CH=CH-; and

R₂ is selected from
- aromatic or aliphatic C₃-C₁₈-hydrocarbon rings, which can optionally be substituted with one or more selected from the group consisting of alkyl, alkenyl, alkynyl, aryl, carboxy, hydroxy, amine, nitro, thiol, sulfoxy, sulfone groups which can optionally be substituted and/or form further rings, and halogen atoms;
- aromatic or aliphatic C₃-C₁₈-heterocycles, which can optionally be substituted with one or more selected from the group consisting of alkyl, alkenyl, alkynyl, aryl, carboxy, hydroxy, amine, nitro, thiol, sulfoxy, sulfone groups which can optionally be substituted and/or form further rings, and halogen atoms;
   comprising reacting a compound of the following formula 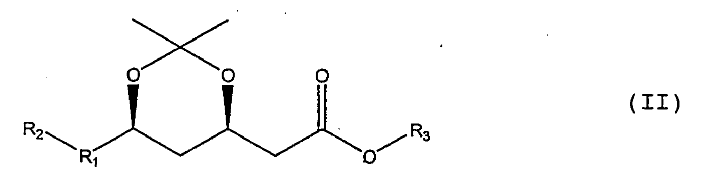
wherein R₁ and R₂ are the same as defined above; and
R₃ is a straight-chain or branched C₁-C₆-alkyl group
with a deprotecting agent in a solvent.

The lactones of formula (I) can form precursors to or backbones of a group of pharmacologically active compounds called statins. Known statins in which the lactones of formula (I) form the backbone include Lovastatin and Simvastatin, known statins for which the lactones of formula (I) form precursors include Atorvastatin, Cerivastatin, Fluvastatin, Pravastatin and Rosuvastatin. The formulas of those compounds and the corresponding R-groups are shown below in table 1.

**Table 1**

| Compound | R₁ | R₂ |
|---|---|---|
| 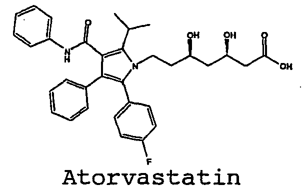 | -CH₂-CH₂- | 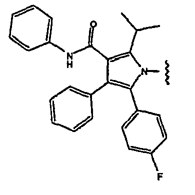 |
| 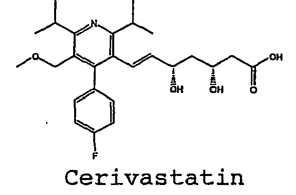 | -CH=CH- | 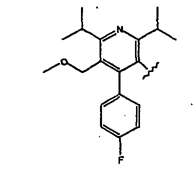 |
| 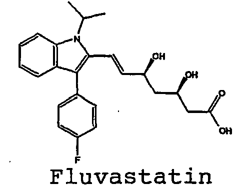 | -CH=CH- | 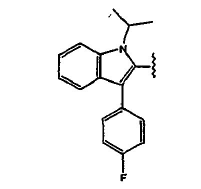 |
| 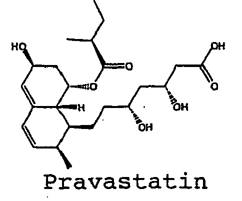 | -CH₂-CH₂- | 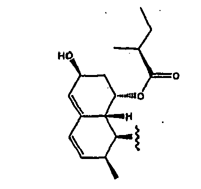 |
| 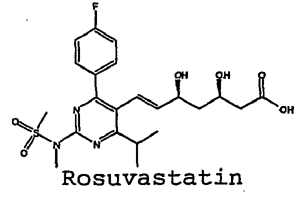 | -CH=CH- | 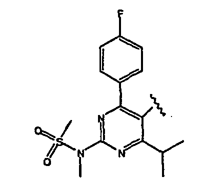 |
| 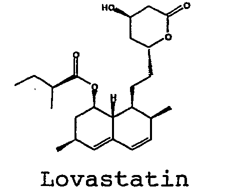 | -CH₂-CH₂- | 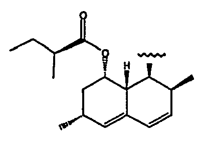 |
| 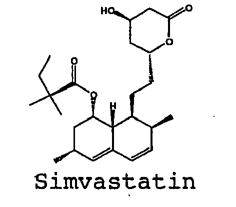 | -CH₂-CH₂- | 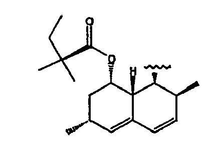 |

Statins are 3-hydroxy-3-methyl-glutaryl-coenzyme A reductase (HMG-CoA reductase) inhibitors and are used to lower cholesterol levels in people at risk from cardiovascular disease because of hypercholesterolemia.

Statins work by inhibiting HMG-CoA reductase, an enzyme in the metabolic pathway for the synthesis of cholesterol. This enzyme catalyses the conversion of HMG-CoA to mevalonate which is the rate determining step in the biosynthesis of cholesterol. A decreased production of cholesterol stimulates low density lipoprotein (LDL) receptors and consequently reduces the concentration of LDL particles in the bloodstream. This reduces the risk of coronary artery disease.

### Lactones of the following formula

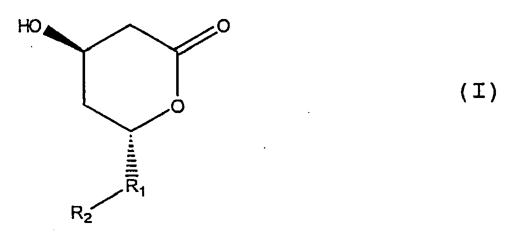

are for example known from the US-patent US 5,280,126 as an intermediate in the synthesis of Atorvastatin.

According to this document the compounds of formula (II) can be first reacted with an acid such as hydrochloric acid in order to remove the acetonide protecting group and then with a base such as sodium hydroxide in order to cleave the ester. It is further disclosed to convert the resulting product to the corresponding lactone of the formula (I).

This method has the problem that it uses potentially large amounts of highly corrosive substances which are difficult to handle and can cause environmental problems.

The use of strong acids such as hydrochloric acid in the deprotection of large organic compounds further carries the risk of degrading some of the starting and/or target compounds leading to a reduced yield.

The reaction further involves a reaction step in an acidic environment followed by a reaction step in a basic environment, followed by a neutralisation and optionally another reaction step in a basic environment.

Such a relatively large number of steps is not only time consuming but also wasteful in resources. Also there is always the problem of the generation of excessive heat associated with acid/base reactions.

It is therefore an object of the invention to describe a new method for producing lactones of formula (I) which can be effected in fewer steps and without the use of strong acids.

It was now surprisingly found that certain hydroxylammonium salts can be successfully used to directly convert the compounds of formula (II) to the lactones of formula (I).

This reaction results in lactones of formula (I) of high purity.

This reaction proceeds in a single step and in the absence of strong mineral acids or bases.

The object is therefore achieved by a method for producing lactones of formula (I) wherein the deprotecting agent is a compound of the following formula 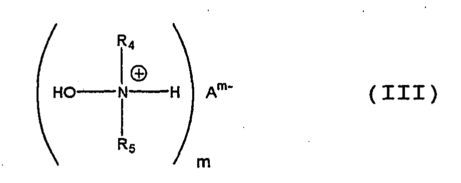 wherein R₄ and R₅ are the same or different and hydrogen or straight-chain or branched C₁-C₆ alkyl groups;
wherein A is an organic or inorganic anion; and
wherein m is an integer.

The method according to this invention involves fewer steps and results in easily purified products in high yields.

The compounds of formula (III) employed are less corrosive and much easier to handle than e.g. strong mineral acids due to their more moderate pH value.

In an embodiment of the invention R₂ comprises a substituted hexahydronaphthyl group, preferably one that is substituted with at least one methyl group and/or at least one butyrate group, which can optionally be substituted with one or more lower alkyl groups.

In another embodiment of the invention R₂ comprises a substituted heterocycle comprising at least one nitrogen atom within the ring system, preferably one that is substituted with at least one 4-fluoro-phenyl group and/or at least one isopropyl group.

Compounds of such a structure are known to show biological activity or act as precursors to compounds which show biological activity and are therefore of interest in the synthesis of active ingredients for various pharmaceuticals.

In an embodiment of the invention the lactone of formula (I) is a compound that shows HMG-CoA reductase inhibitory activity. Preferably it is selected from the group consisting of Lovastatin and Simvastatin.

Such compounds can be used to lower LDL cholesterol and reduce the risk of coronary artery disease and are therefore of high commercial interest.

In an embodiment of the invention the method further comprises the step of treating the lactone of formula (I) with a metal hydroxide in order to obtain a compound of the following formula 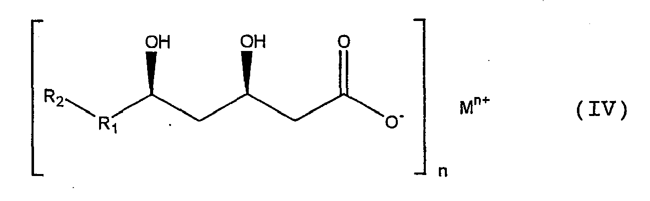 wherein R₁ and R₂ are defined as above;
wherein M is any metal; and
wherein n is an integer.

Preferably the compound of formula (IV) is a compound that shows HMG-CoA reductase inhibitory activity, especially one selected from the group consisting of the metal salts of Atorvastatin, Cerivastatin, Fluvastatin, Rosuvastatin and Pravastatin.

The compounds of formula (IV) are known to show biological activity and are therefore of interest in the synthesis of active ingredients various pharmaceuticals. Compounds showing HMG-GoA reductase inhibitory activity in general and the metal salts of Atorvastatin, Cerivastatin, Fluvastatin, Pravastatin and Rosuvastatin in particular show powerful LDL cholesterol lowering activity and can be used to prevent coronary artery disease and are therefore of commercial interest.

In an embodiment of the invention the metal hydroxide is an alkali metal hydroxide, especially sodium hydroxide.

Sodium hydroxide is a preferred agent for the treatment of the lactones of formula (I) since it is a relatively cheap and easily available agent and at the same time results in an easily purified product which if needed can be further reacted on in order to form e.g. Atorvastatin calcium trihydrate.

In a further embodiment of the invention the compound of formula (IV) is a metal salt of Atorvastatin. Preferably the method further comprises the step of converting the metal salt of Atorvastatin into a calcium salt of Atorvastatin.

Atorvastatin calcium trihydrate is a particularly powerful LDL cholesterol lowering compound and therefore of particular commercial interest.

Although it is possible to directly treat the lactones of formula (I) with calcium hydroxide in order to form the desired calcium salt of Atorvastatin, it has been shown that it is often more effective to first convert the lactones of formula (I) to a different metal salt of Atorvastatin such as the sodium salt of Atorvastatin and then convert it to the calcium salt since calcium hydroxide shows a relatively low basicity compared to for example sodium hydroxide and is therefore not as effective in cleaving the lactone ring.

In an embodiment of the above measure the step of converting the metal salt of Atorvastatin into a calcium salt of Atorvastatin comprises treating the metal salt of Atorvastatin with a calcium salt, especially a calcium salt selected from the group consisting of CaCl₂ and Ca(OAc)₂.

This method has been shown to give improved yields over a direct reaction of the lactone with calcium hydroxide.

In a further embodiment of the invention R₃ is a *t*-butyl group.

A *t*-butyl group is particularly easy to remove substantially facilitating liberation of the free acid.

In a further embodiment of the invention R₄ and R₅ are both hydrogen.

A is preferably an anion of a mineral acid. More preferably the compound of formula (III) is selected from the group consisting of hydroxylammonium sulfates and hydroxylammonium chlorides.

Compounds of formula (III) in which R₄ and R₅ are both hydrogen and/or A is a monovalent anion of a mineral acid in general and hydroxylammonium sulfates and hydroxylammonium chlorides in particular have been shown to give good results in the reaction with the compound of formula (II). They are also relatively inexpensive and readily available compounds making them well suited to large scale use.

In a further embodiment of the invention the solvent comprises a mixture of solvents, especially a mixture of solvents comprising at least one C₁-C₆ alcohol, at least one lower liquid ketone and water. The at least one C₁-C₆ alcohol is thereby preferably selected from the group consisting of methanol, ethanol, isopropanol. The at least one lower liquid ketone is thereby preferably selected from the group consisting of acetone, methyl ethyl ketone and methyl isobutyl ketone, and is most preferably acetone.

Preferably the mixture of solvents comprises 10-15% by volume of water, especially 12-13% by volume of water.

Preferably the mixture of solvents comprises 25-30% by volume of the at least one lower liquid ketone, especially 27-28% by volume of the at least one lower liquid ketone.

Such mixtures of solvents have been proven to be particularly conductive to the reactions according to the invention since they strike a good balance between the solubility of the relatively unpolar compounds of formula (II) and the more polar compounds of formula (III). It is also been shown that this mixture is advantageous since the ketone can relatively easily be removed therefore arriving at a mixture of solvents which is particularly suitable for the preparation of e.g. Atorvastatin calcium trihydrate.

In an embodiment of the invention the compound of formula (II) is reacted with the compound of formula (III) at a temperature from 20-85°C, preferably 40-75°C, and especially 60-70°C.

In an embodiment of the invention the lactone of formula (I) is treated with a metal hydroxide at a temperature from 20-85°C, preferably 40-75°C and especially 60-70°C.

When reacting larger and potentially unstable organic compounds such as those of formula (I) or formula (II) a balance must be found between the fact that at higher temperatures these compounds have the tendency to degrade, resulting in lower yields and the necessity that the temperature is high enough so that the reactions proceed within a reasonable period of time. This must especially be seen with regards to potential solubility problems for example of the metal hydroxide.

It has been found that in the above named temperature ranges the reactions can be performed in under one hour while obtaining good yields.

In a further embodiment of the invention the lactone of formula (I) is treated with a metal hydroxide at a pH from 11.5 to 12.0.

A pH value above pH 12 should be avoided because an access of metal hydroxide can, if the metal salt is further treated in order to form e.g. Atorvastatin calcium trihydrate, cause the formation of high amounts of calcium hydroxide which makes the purification of the desired product more difficult. At to low a pH-value the lactone of formula (I) will not be cleaved effectively.

In an embodiment of the invention all steps are performed without isolating any intermediates.

Not isolating any intermediates speeds up the process therefore making it more economical. It is also ecologically more benign since there is no need to use extra agents or other materials in order to isolate the intermediates therefore saving on resources.

It is understood that the above features and the features described below can be used not only in the described combination but also in other combinations or in isolation without departing from the scope of the invention.

The invention is now illustrated by means of examples. These examples are not intended to limit the scope of the invention in any way.

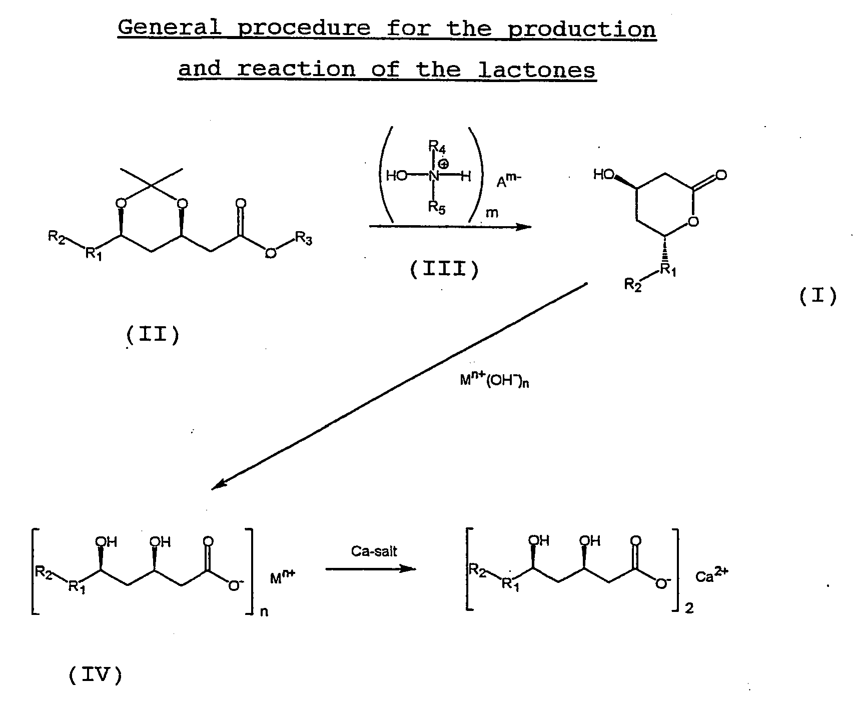

A compound of formula (II) is dissolved in a solvent and stirred together with the compound of formula (III) at 25-80°C. The progression of the reaction is monitored by HPLC. After most of the starting compound of formula (II) has been consumed, usually after about one hour, the stirring is stopped and the lactone of formula (I) is isolated.

Optionally the temperature of the reaction medium is adjusted to 25-80°C and a metal hydroxide is added. The resulting mixture is kept stirring for 30 minutes to 1 hour. At the end of this period of time the pH is measured and should be at 11.5 to 12.

Optionally now about half of the solvent mixture can be distilled off and the removed solvent can be replaced by the same amount of a C₁-C₆ alcohol. The resulting mixture is then heated to 60-70°C and a calcium salt is added. The resulting mixture is stirred for about 30 minutes.

The resulting turbid reaction mixture is filtered to remove any precipitated calcium hydroxide. The clear filtrate is heated to 60-70°C and water is added slowly to the reaction mixture. The calcium salt starts to precipitate as white crystals. After the end of the water addition the resulting slurry is stirred over night at room temperature. The desired end product is collected by filtration and dried at an elevated temperature under vacuum.

### EXAMPLE 1

### Preparation of Atorvastatin calcium trihydrate from a protected t-butyl ester of Atorvastatin

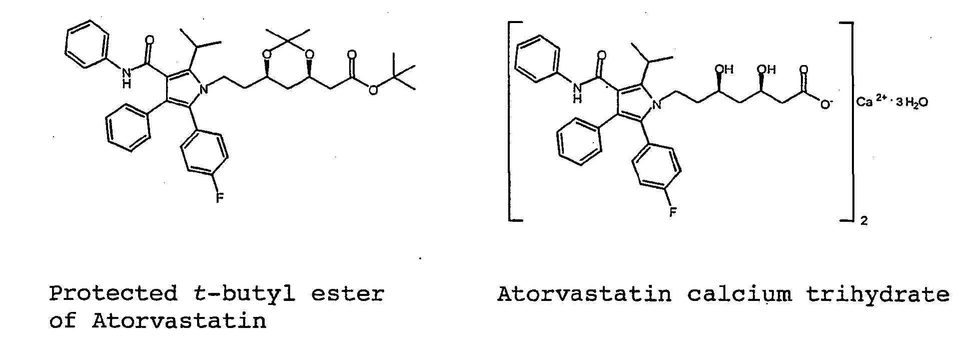

A two liter three-neck glass reactor equipped with a reflux condenser, a thermometer and a mechanical stirrer was charged with 325 ml methanol, 150 ml acetone and 75 ml of water. 50 g of the protected *t*-butyl ester of Atorvastatin and 17.5 g hydroxylammonium chloride were added and the mixture was heated to reflux. The temperature measured at reflux was 67°C. After one hour a sample was taken from the colorless reaction mixture and analyzed by HPLC. The analysis indicated that the Atorvastatin lactone had been formed with a relative purity of 99.9%.

25 ml of a 37.5% w/w NaOH solution were added to the reaction mixture. The temperature of the reaction mixture was adjusted to 60°C and stirring was continued for 40 minutes at this temperature. At this point the pH value of the solution was measured as 12.

About 240 ml of the solvent mixture were distilled off and 240 ml methanol were added to the reactor. The temperature of the solution was adjusted to 65°C, 6 g CaCl₂·2H₂O were added and the resulting mixture was stirred for half an hour at 65-68°C. The resulting turbid solution was filtered over celite to remove the precipitated Ca(OH)₂.

The resulting clear filtrate was heated to 65-70°C and 720 ml water were added over a period of 3 hours while maintaining the above temperature. When about half the water had been added Atorvastatin calcium started to precipitate as white crystals. After the end of the water addition the heating was stopped and the resulting slurry was left stirring overnight at room temperature. The obtained slurry was filtered and the filter cake was washed with 200 ml of water. The obtained white crystals were dried under vacuum at 60°C to constant weight to yield 28.6 g (75%) of the desired product.

### EXAMPLE 2

### Preparation of Atorvastatin calcium trihydrate from a protected t-butyl ester of Atorvastatin

A 1 1 three-neck glass reactor equipped with a reflux condenser, a thermometer and a mechanical stirrer was charged with 130 ml isopropyl alcohol, 60 ml acetone and 30 ml water. 20 g of the protected *t*-butyl ester of Atorvastatin and 70 g hydroxylammonium chloride were added and the mixture was heated to 67°C. After about one hour a sample was taken from the colorless reaction mixture and analyzed by HPLC. The analysis indicated that the Atorvastatin lactone had been formed with a relative purity of 99.6%.

10 ml of a 37.5% w/w NaOH solution were added to the reaction mixture. The temperature of the reaction mixture was adjusted to 65°C and stirring continued for 45 minutes. At this point the pH of the solution was measured as 11.9.

About 95 ml of the solvent mixture were distilled off and 95 ml isopropyl alcohol were added. The temperature of the reaction mixture was adjusted to 65°C, 2.4 g of CaCl₂·2H₂O were added, and the resulting mixture was stirred for 40 minutes at 65-70°C. The resulting turbid solution was filtered over celite to remove the precipitated Ca(OH)₂.

The resulting clear filtrate was transferred to a crystallization flask and heated to 70°C. 285 ml water were added to the flask over a period of 3 hours while maintaining the above temperature. The heating was stopped and the resulting mixture was stirred overnight at room temperature resulting in a white precipitate. The white precipitate was collected by filtration and washed with 100 ml water. The so-obtained white crystals were dried at 60°C under vacuum to constant weight to yield 13.7 g (75%) of Atorvastatin calcium trihydrate.

## Claims

1. Method for producing lactones of the following formula 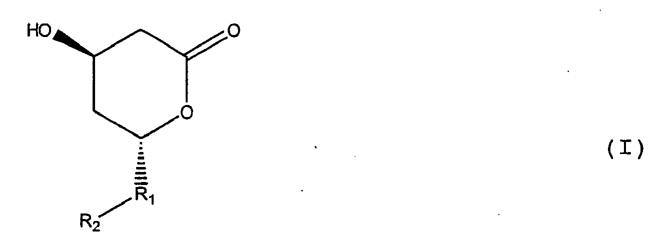 wherein
R₁ is -CH₂-CH₂- or -CH=CH-; and
R₂ is selected from
- aromatic or aliphatic C₃-C₁₈-hydrocarbon rings, which can optionally be substituted with one or more selected from the group consisting of alkyl, alkenyl, alkynyl, aryl, carboxy, hydroxy, amine, nitro, thiol, sulfoxy, sulfone groups which can optionally be substituted and/or form further rings, and halogen atoms;
- aromatic or aliphatic C₃-C₁₈-heterocycles, which can optionally be substituted with one or more selected from the group consisting of alkyl, alkenyl, alkynyl, aryl, carboxy, hydroxy, amine, nitro, thiol, sulfoxy, sulfone groups which can optionally be substituted and/or form further rings, and halogen atoms;
comprising reacting a compound of the following formula 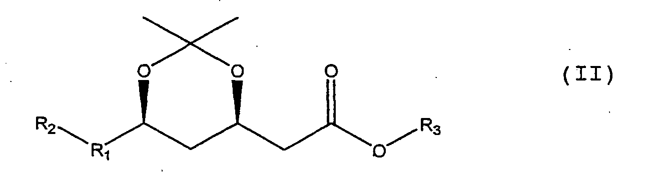
wherein R₁ and R₂ are the same as defined above; and
R₃ is a straight-chain or branched C₁-C₆-alkyl group
with a deprotecting agent in a solvent,
**characterized in that** the deprotecting agent is a compound of the following formula 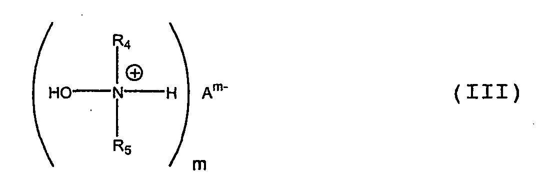 wherein R₄ and R₅ are the same or different and hydrogen or straight-chain or branched C₁-C₆-alkyl groups;
wherein A is an organic or inorganic anion; and
wherein m is an integer.

2. Method according to claim 1, **characterized in that** R₂ comprises a substituted hexahydronaphthyl group.

3. Method according to claim 2, **characterized in that** the substituted hexahydronaphthyl group is substituted with at least one methyl group.

4. Method according to claim 2 or 3, **characterized in that** the substituted hexahydronaphthyl group is substituted with at least one butyrate group, which can optionally be substituted with one or more lower alkyl groups.

5. Method according to any one of claim 1 to 4, **characterized in that** the lactone of formula (I) is a compound that shows HMG-CoA reductase inhibitory activity.

6. Method according to claim 1 to 5, **characterized in that** the lactone of formula (I) is selected from the group consisting of Lovastatin and Simvastatin.

7. Method according to claim 1, **characterized in that** R₂ comprises a substituted heterocycle comprising at least one nitrogen atom within the ring system.

8. Method according to claim 7, **characterized in that** the substituted heterocycle comprising at least one nitrogen atom in the ring system is substituted with at least one 4-fluoro-phenyl group.

9. Method according to claim 7 or 8, **characterized in that** the substituted heterocycle comprising at least one nitrogen atom in the ring system is substituted with at least one isopropyl group

10. Method according to anyone of claims 1 to 9, **characterized in that** it further comprises the step of treating the lactone of formula (I) with a metal hydroxide in order to obtain a compound of the following formula 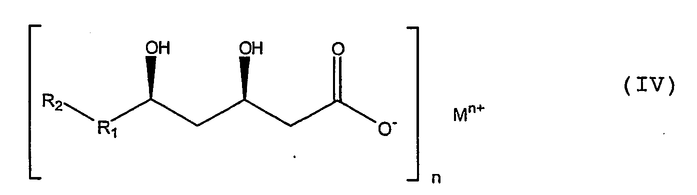 wherein R₁ and R₂ are the same as defined above;
wherein M is any metal; and
wherein n is an integer.

11. Method according to claim 10, **characterized in that** the compound of formula (IV) is a compound that shows HMG-CoA reductase inhibitory activity.

12. Method according to claim 10 or 11, **characterized in that** the compound of formula (IV) is selected from the group consisting of the metal salts of Atorvastatin, Cerivastatin, Fluvastatin, Pravastatin and Rosuvastatin.

13. Method according to anyone of claims 10 to 12, **characterized in that** the metal hydroxide is an alkali metal hydroxide.

14. Method according to claim 13, **characterized in that** the alkali metal hydroxide is sodium hydroxide.

15. Method according to anyone of claims 10 to 14, **characterized in that** the compound of formula (IV) is a metal salt of Atorvastatin.

16. Method according to claim 15, **characterized in that** it further comprises the step of converting the metal salt of Atorvastatin into a calcium salt of Atorvastatin.

17. Method according to claim 16, **characterized in that** the step of converting the metal salt of Atorvastatin into a calcium salt of Atorvastatin comprises treating the metal salt of Atorvastatin with a calcium salt.

18. Method according to claim 17, **characterized in that** the calcium salt is selected from the group consisting of CaCl₂ and Ca(OAc)₂.

19. Method according to any one of claims 1 to 18 **characterized in that** R₃ is a *t*-butyl group.

20. Method according to any one of claims 1 to 19, **characterized in that** R₄ and R₅ are both hydrogen.

21. Method according to any one of claims 1 to 20, **characterized in that** A is an anion of a mineral acid.

22. Method according to claim 21, **characterized in that** the compound of formula (III) is selected from the group consisting of hydroxylammonium sulfates and hydroxylammonium chlorides.

23. Method according to any one of claims 1 to 22, **characterized in that** the solvent comprises a mixture of solvents.

24. Method according to claim 23, **characterized in that** the mixture of solvents comprises at least one C₁-C₆ alcohol, at least one lower liquid ketone and water.

25. Method according to claim 24, **characterized in that** the at least one C₁-C₆ alcohol is selected from the group consisting of methanol, ethanol and isopropanol.

26. Method according to 24 or 25, **characterized in that** the at least one lower liquid ketone is selected from the group consisting of acetone, methyl ethyl ketone and methyl isobutyl ketone.

27. Method according to claim 26, **characterized in that** the at least one lower liquid ketone is acetone.

28. Method according to any one of claims 23 to 27**, characterized in that** the mixture of solvents comprises 10-15% by volume of water.

29. Method according to claim 28, **characterized in that** the mixture of solvents comprises 12-13% by volume of water.

30. Method according to any one of claims 23 to 29, **characterized in that** the mixture of solvents comprises 25-30% by volume of at least one lower liquid ketone.

31. Method according to claim 30, **characterized in that** the mixture of solvents comprises 27-28% by volume of the at least one lower liquid ketone.

32. Method according to any one of claims 1 to 31, **characterized in that** the compound of formula (II) is reacted with the compound of formula (III) at a temperature from 20°C to 85°C.

33. Method according to claim 32, **characterized in that** the compound of formula (II) is reacted with the compound of formula (III) at a temperature from 40°C to 75°C.

34. Method according to claim 33, **characterized in that** the compound of formula (II) is reacted with the compound of formula (III) at a temperature from 60°C to 70°C.

35. Method according to any one of claims 10 to 34, **characterized in that** the lactone of formula (I) is treated with a metal hydroxide at a temperature from 20°C to 85°C.

36. Method according to claim 33, **characterized in that** the lactone of formula (I) is treated with a metal hydroxide at a temperature from 40°C to 75°C.

37. Method according to claim 36, **characterized in that** the lactone of formula (I) is treated with a metal hydroxide at a temperature from 60°C to 70°C.

38. Method according to any one of claims 10 to 37, **characterized in that** the lactone of formula (*I*) is treated with a metal hydroxide at a pH from 11.5 to 12.0.

39. Method according any one of claims 1 to 38, **characterized in that** all steps are performed without isolating any intermediates.

## Patentansprüche

1. Die untenstehende Formel ist eine Formel zur Laktonherstellung. 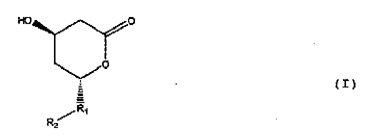 in dieser Formel bedeutet
Ri = -CH₂-CH₂- oder -CH=CH-.
R₂ wird aus den untenstehenden Komponenten ausgewählt
- aromatische oder alifatische C₃-C₁₈-Kohlenwasserstoffringe, die optional substituieren können und/oder andere Ringe und Halogenausscheidungen bilden können und mit einer oder mehreren Komponenten der Gruppe optional substituieren können, die aus Alkyl, Alkenyl, Aryl, Carboxy, Hydroxy, Amin, Nitro, Thyol, Sulfoxy, Sulfon-Gruppen besteht;
- aromatische oder alifatische C3-C18-Heteroringe, die optional substituieren und/oder andere Ringe und Halogenausscheidungen bilden können und mit einer oder mehreren Komponenten der Gruppe optional substituieren können, die aus Alkyl, Alkenyl, Aryl, Carboxy, Hydroxy, Amin, Nitro, Thyol, Sulfoxy, Sulfon-Gruppen besteht;
und entsteht aus der Reaktion der Verbindung der untenstehenden Formel. 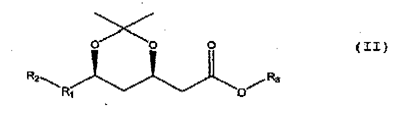 hier haben R₁ und R₂ die oben genannte Bedeutung,
R₃ ist hingegen eine geradkettige oder verzweigte C₁-C₆-Alkylgruppe,
in einem Lösungsmittel befindet sich ein Trenner, der eine Verbindung der untenstehenden Formel ist, 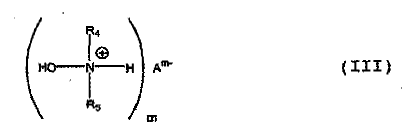 in dieser Formel sind R₄ und R₅ eine gleiche oder ungleiche, hydrogene oder geradkettige oder verzweigte C₁-C₆-Alkylgruppe,
in dieser Formel ist A ein organisches oder anorganisches Anion und M eine Ganzzahl.

2. Verfahren nach Anspruch 1, **dadurch gekennzeichnet, dass** R₂ eine substituierte Hexahydronaphthyl-Grupppe enthält.

3. Verfahren nach Anspruch 2, **dadurch gekennzeichnet, dass** die substituierte Hexahydronaphthyl-Gruppe durch mindestens eine Methylgruppe substituiert ist.

4. Verfahren nach Anspruche 2 oder 3, **dadurch gekennzeichnet, dass** die substituierte Hexahydronaphthyl-Gruppe optional durch mindestens eine Butyrat-Gruppe substituiert ist, die durch eine oder eine geringere niedere Alkyl-Gruppe substituiert sein kann.

5. Verfahrens nach einem der Ansprüche 1 bis 4, **dadurch gekennzeichnet, dass** das Lakton der Formel (I) eine Verbindung ist, die die Aktivität des HMG-CoA Reduktase-Hemmers anzeigt.

6. Verfahrens nach Ansprüche 1 bis 5, **dadurch gekennzeichnet, dass** das Lakton der Formel (I) aus der Gruppe ausgewählt wurde, die Lovastatin und Simvastatin enthält.

7. Verfahren nach Anspruch 1, **dadurch gekennzeichnet, dass** das R₂-Ringsystem einen substituierten Heteroring aus mindestens einem Stickstoffatom enthält.

8. Verfahren nach Anspruch 7, **dadurch gekennzeichnet, dass** im Ringsystem der substituierte Heteroring, der aus mindestens einem Stickstoffatom besteht, durch mindestens eine 4-Fluorphenyl-Gruppe substituiert ist.

9. Verfahren nach Anspruche 7 oder 8, **dadurch gekennzeichnet, dass** im Ringsystem der substituierte Heteroring, der aus mindestens einem Stickstoffatom besteht, durch mindestens eine Isopropylgruppe substituiert ist.

10. Verfahrens nach einem der Ansprüche 1 bis 9, **dadurch gekennzeichnet, dass** sich das Lakton der Formel (I) mit einem Metallhydroxid verbindet, um so eine Verbindung der Formel zu erhalten. 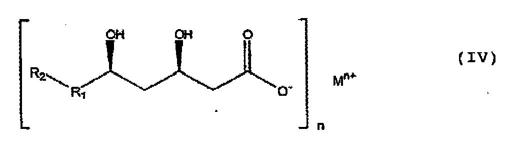 Hier haben R₁ und R₂ die oben genannte Bedeutung.
Hier ist M ein beliebiges Metall und n eine Ganzzahl.

11. Verfahren nach Anspruch 10, **dadurch gekennzeichnet, dass** die Verbindung der Formel (IV) eine Verbindung ist, die die Aktivität des HMG-CoA Reduktase-Hemmers anzeigt.

12. Verfahren nach Anspruch 10 oder 11, **dadurch gekennzeichnet, dass** die Verbindung der Formel (IV) aus der Gruppe ausgewählt wird, die die Metallsalze Atorvastatin, Cerivastatin, Fluvastatin, Pravastatin und Rosuvastatin enthält.

13. Verfahrens nach einem der Ansprüche 10 bis 12, **dadurch gekennzeichnet, dass** das Metallhydroxid ein alkalisches Metallhydroxid ist.

14. Verfahren nach Anspruch 13, **dadurch gekennzeichnet, dass** das alkalische Metallhydroxid ein Natriumhydroxid ist.

15. Verfahrens nach einem der Ansprüche 10 bis 14, **dadurch gekennzeichnet, dass** das Atorvastatin der Verbindung der Formel (IV) ein Metallsalz ist.

16. Verfahren nach Anspruch 15, **dadurch gekennzeichnet, dass** es auch den Schritt zur Überführung des Atorvastatin Metallsalzes in das Atorvastatin Calciumsalz umfasst.

17. Verfahren nach Anspruch 16, **dadurch gekennzeichnet, dass** der Schritt zur Überführung des Atorvastatin Metallsalzes in das Calciumsalz von Atorvastatin durch Reaktion des Metallsalzes von Atorvastatin mit Calciumsalz erfolgt.

18. Verfahren nach Anspruch 17, **dadurch gekennzeichnet, dass** das Calciumsalz aus einer Gruppe ausgewählt wurde, die CaCl₂ und Ca(OAc)₂ enthält.

19. Verfahrens nach einem der Ansprüche 1 bis 18, **dadurch gekennzeichnet, dass** R₃ eine Butyl-Gruppe ist.

20. Verfahrens nach einem der Ansprüche 1 bis 19, **dadurch gekennzeichnet, dass** R₄ und R₅ beide Wasserstoff sind.

21. Verfahrens nach einem der Ansprüche 1 oder 20, **dadurch gekennzeichnet, dass** A ein Anion der Mineralsäure ist.

22. Verfahren nach Anspruch 21, **dadurch gekennzeichnet, dass** die Verbindung der Formel (III) aus der Gruppe ausgewählt wurde, die Hydroxylammoniumsulfate und Hydroxylammoniumchloride enthält.

23. Verfahrens nach einem der Ansprüche 1 bis 22, **dadurch gekennzeichnet, dass** das Lösungsmittel aus einem Lösungsmittelgemisch besteht.

24. Verfahren nach Anspruch 23, **dadurch gekennzeichnet, dass** das Lösungsmittelgemisch aus mindestens einem *C₁*-*C₆* Alkohol, mindestens einem niederen flüssigen Keton und Wasser besteht.

25. Verfahren nach Anspruch 24, **dadurch gekennzeichnet, dass** es aus einer Gruppe ausgewählt wurde, die mindestens einen *C₁-C₆* Alkohol, Ethanol und Isopropanol enthält.

26. Verfahren nach Anspruch 24 oder 25, **dadurch gekennzeichnet, dass** mindestens ein niederes flüssiges Keton aus einer Gruppe ausgewählt wurde, die Aceton, Methylethylketon und Methylisobuthylketon enthält.

27. Verfahren nach Anspruch 26, **dadurch gekennzeichnet, dass** mindestens ein niederes flüssiges Keton ein Aceton ist.

28. Verfahrens nach einem der Ansprüche 23 bis 27, **dadurch gekennzeichnet, dass** das Volumen des Lösungsmittelgemischs bis zu 10-15 % aus Wasser besteht.

29. Verfahren nach Anspruch 28, **dadurch gekennzeichnet, dass** das Volumen des Lösungsmittelgemischs bis zu 12-13 % aus Wasser besteht.

30. Verfahrens nach einem der Ansprüche 23 bis 29, **dadurch gekennzeichnet, dass** das Volumen des Lösungsmittelgemischs bis zu 25-30 % aus mindestens einem niederen flüssigen Keton besteht.

31. Verfahren nach Anspruch 30, **dadurch gekennzeichnet, dass** das Volumen des Lösungsmittelgemischs bis zu 27-28 % aus mindestens einem niederen flüssigen Keton besteht.

32. Verfahrens nach einem der Ansprüche 1 bis 31**, dadurch gekennzeichnet, dass** die Verbindung der Formel (II) bei einer Temperatur von 20°C bis 85°C mit der Verbindung der Formel (III) eine Reaktion eingeht.

33. Verfahren nach Anspruch 32, **dadurch gekennzeichnet, dass** die Verbindung der Formel (II) bei einer Temperatur von 40°C bis 75°C mit der Verbindung der Formel (III) eine Reaktion eingeht.

34. Verfahren nach Anspruch 33, **dadurch gekennzeichnet, dass** die Verbindung der Formel (II) bei einer Temperatur von 60°C bis 70°C mit der Verbindung der Formel (III) eine Reaktion eingeht.

35. Verfahrens nach einem der Ansprüche 10 bis 34, **dadurch gekennzeichnet, dass** das Lakton der Formel (I) bei einer Temperatur von 20°C bis 85°C mit einem Metallhydroxid eine Reaktion eingeht.

36. Verfahren nach Anspruch 33, **dadurch gekennzeichnet, dass** das Lakton der Formel (I) bei einer Temperatur von 40°C bis 75°C mit einem Metallhydroxid eine Reaktion eingeht.

37. Verfahren nach Anspruch 36, **dadurch gekennzeichnet, dass** das Lakton der Formel (I) bei einer Temperatur von 60°C bis 70°C mit einem Metallhydroxid eine Reaktion eingeht.

38. Verfahrens nach einem der Ansprüche 10 bis 37, **dadurch gekennzeichnet, dass** das Lakton der Formel (I) bei einem pH-Wert von 11.5 bis 12.0 mit einem Metallhydroxid eine Reaktion eingeht.

39. Verfahrens nach einem der Ansprüche 1 bis 38, **dadurch gekennzeichnet, dass** alle Schritte ohne Isolierung eines Zwischenproduktes durchgeführt werden.

## Revendications

1. Méthode pour produire lactones de la formule suivante 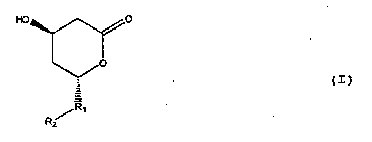 où
R₁ est -CH₂-CH₂- ou -CH=CH-; et
R₂ est sélectionné de
- C₃-C₁₈-hydrocarbure anneaux aromatiques ou aliphatiques, qui peuvent facultativement être substitués par un(e) ou plus être sélectionné(e)(s) dans le groupe constitué du groupe de l'alkyle, l'alkenyle, l'alkynyle, l'aryle, la carboxy, l'hydoxy, l'amine, du nitro, du thiol, la sulfoxy, la sulfone qui peuvent facultativement être substitués et/ou forment encore des anneaux, et des atomes halogènes ;
- C₃-C₁₈-hétérocycles aromatiques ou aliphatiques, qui peuvent facultativement être substitués par un(e) ou plus être selectionné(e)(s) dans le groupe constitué du groupe de l'alkyle, l'alkenyle, l'alkynyle, l'aryle, la carboxy, l'hydoxy, l'amine, du nitro, du thiol, la sulfoxy, la sulfone qui peuvent facultativement être substitués et/ou forment encore des anneaux, et des atomes halogènes ;
comportant réagir un composé de la formule suivante 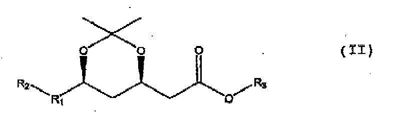
où R₁ et R₂ sont les mêmes comme définis ci-dessus
R3 est une chaîne droite ou bien un groupe d'alkyle C₁-C₆ ramifié
avec un agent déprotecteur dans un solvant,
**caractérisé par le fait que** l'agent déprotecteur est un composé de la formule suivante 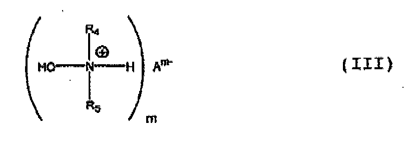 où R₄ et R₅ sont les mêmes ou bien différents et hydrogène ou bien
une chaîne droite ou bien des groupes alkyles C₁-C₆ ramifiés ;
où A est un anion organique ou inorganique; et
où m est un entier relatif.

2. Méthode selon le revendication 1, **caractérisé par le fait que** R₂ comporte un groupe d'hexahydronaphthyl substitué.

3. Méthode selon le revendication 2, **caractérisé par le fait que** le groupe d'hexahydronaphthyl substitué est substitué par au moins un groupe de méthyle.

4. Méthode selon le revendication 2 ou 3, **caractérisé par le fait que** le groupe d'hexahydronaphthyl substitué est substitué par au moins un group de butyrate, qui peut facultativement être substitué par un(e) ou plus des groupes d'alkyles inférieurs.

5. Méthode selon l'une quelconque des revendication 1 à 4, **caractérisé par le fait que** la lactone de la formule (I) est un composé qui présente l'effet inhibiteur d'HMG-CoA-réductase

6. Méthode selon la revendication 1 à 5, **caractérisée par le fait que** la lactone de la formule (I) est sélectionné dans le groupe composé de la Lovastatine et la Simvastatine.

7. Méthode selon le revendication 1, **caractérisé par le fait que** R₂ comporte un hétérocycle substitué comportant au moins un nitrogène à l'intérieur du système d'anneaux.

8. Méthode selon le revendication 7, **caractérisé par le fait que** l'hétérocycle substitué comportant au moins un atome nitrogène dans le système d'anneaux est substitué par au moins d'un groupe de 4-fluoro-phényle.

9. Méthode selon le revendication 7 ou 8, **caractérisé par le fait que** l'hétérocycle substitué comportant au moins un atome nitrogène dans le système d'anneaux est substitué par au moins d'un groupe d'iso-propyle.

10. Méthode selon l'une quelconque des revendication 1 à 9, **caractérisé par le fait qu'**elle comporte encore l'étape de traiter la lactone de la formule (I) avec un hydroxyde de métal afin que obtenir un composé de la formule 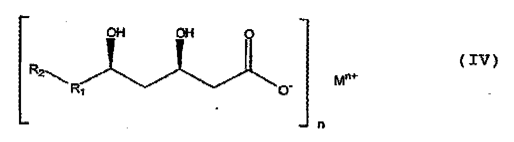 où R₁ et R₂ sont les mêmes comme définis ci-dessus ;
où M est un métal, n'importe lequel; et
où n est un entier relatif.

11. Méthode selon le revendication 10, **caractérisé par le fait que** le composé de la formule (IV) est un composé qui présente l'effet inhibiteur d'HMG-CoA-réductase

12. Méthode selon le revendication 10 ou 11, **caractérisé par le fait que** le composé de la formule (IV) est sélectionné dans le group constitué des sels métalliques de l'Atorvastatin, le Cerivastatin, le Flu-vastatine, le Pravastatine et le Rosuvastatine.

13. Méthode selon le revendication 10 ou 12, **caractérisé par le fait que** l'hydroxyde de métal est un hydroxyde du métal alcalin.

14. Méthode selon le revendication 13, **caractérisé par le fait que** l'hydroxyde de métal est l'hydroxyde de sodium.

15. Méthode selon l'une quelconque des revendication 10 à 14, **caractérisé par le fait que** le composé de la formule (IV) est un sel métallique de l'Atorvastatine.

16. Méthode selon le revendication 15, **caractérisé par le fait qu'**elle comporte encore l'étape de transformer le sel métallique de l'Atorvastatine en un sel de calcium de l'Atorvastatine.

17. Méthode selon le revendication 16, **caractérisé par le fait que** l'étape de transformer le sel métallique de l'Atorvastatine en un sel de calcium de l'Atorvastatine comporte traiter le sel métallique de l'Atorvastatine par un sel de calcium.

18. Méthode selon le revendication 17, **caractérisé par le fait que** le sel de calcium est sélectionné dans le group constitué de CaCl₂ et Ca(OAc)₂.

19. Méthode selon l'une quelconque des revendication 1 à 18, **caractérisé par le fait que** R3 est un groupe de t-butyle.

20. Méthode selon l'une quelconque des revendication 1 à 19, **caractérisé par le fait que** R4 et R5 sont tous les deux, hydrogène.

21. Méthode selon l'une quelconque des revendication 1 à 20, **caractérisé par le fait que** A est un anion d'un acide minéral.

22. Méthode selon le revendication 21, **caractérisé par le fait que** le composé de la formule (III) est sélectionné dans le groupe constitué des sulfates de l'hydroxyle d'ammonium et des chlorure de l'hydroxyle d'ammonium.

23. Méthode selon l'une quelconque des revendication 1 à 22, **caractérisé par le fait que** le solvant comporte un mélange des solvants.

24. Méthode selon le revendication 23, **caractérisé par le fait que** le mélange des solvants comporte au moins d'un alcool de *C₁-C₆*, au moins d'une cétone liquide inférieure et d'eau.

25. Méthode selon le revendication 24, **caractérisé par le fait qu'**au moins un alcool de C₁-C₆ est sélectionné dans le groupe constitué du méthanol, l'éthanol et l'isopropanol.

26. Méthode selon le revendication 24 ou 25, **caractérisé par le fait qu'**au moins une cétone liquide inférieure est sélectionné dans le groupe constitué de l'acétone, de la méthyle éthyle cétone et de la méthyle isobutyle cétone.

27. Méthode selon le revendication 26, **caractérisée par le fait qu'**au moins une cétone liquide inférieur est l'acétone.

28. Méthode selon l'une quelconque des revendication 23 à 27, **caractérisé par le fait que** le mélange des solvants comporte 10-15 % de l'eau au volume.

29. Méthode selon le revendication 28, **caractérisé par le fait que** le mélange des solvants comporte 12-13 % de l'eau au volume.

30. Méthode selon l'une quelconque des revendication 23 à 29, **caractérisé par le fait que** le mélange des solvants comporte 25-30% au volume d'au moins une cétone liquide inférieur.

31. Méthode selon le revendication 30, **caractérisé par le fait que** le mélange des solvants comporte 27-28% au volume d'au moins une cétone liquide inférieur.

32. Méthode selon l'une quelconque des revendication 1 à 31, **caractérisé par le fait que** le composé de la formule (II) est réagi avec le composé de la formule (III) à une température de 20°C à 85°C.

33. Méthode selon le revendication 32, **caractérisé par le fait que** le composé de la formule (II) est réagi avec le composé de la formule (III) à une température de 40°C à 75°C.

34. Méthode selon le revendication 33, **caractérisé par le fait que** le composé de la formule (II) est réagi avec le composé de la formule (III) à une temperature de 60°C à 70°C.

35. Méthode selon l'une quelconque des revendication 10 à 34, **caractérisé par le fait que** la lactone de la formule (I) est traité par l'hydroxyde de métal à une température de 20°C à 85°C.

36. Méthode selon le revendication 33, **caractérisé par le fait que** la lactone de la formule (I) est traité par l'hydroxyde de métal à une temperature de 40°C à 75°C.

37. Méthode selon le revendication 36, **caractérisé par le fait que** la lactone de la formule (I) est traité par l'hydroxyde de métal à une temperature de 60°C à 70°C.

38. Méthode selon l'une quelconque des revendication 10 à 37, **caractérisé par le fait que** la lactone de la formule (I) est traité par l'hydroxyde de métal à un pH de 11.5 à 12.0.

39. Méthode selon l'une quelconque des revendication 1 à 38, **caractérisé par le fait que** toutes les étapes sont accomplis sans isolation d'aucun intermédiaire.
